# EUROPEAN PATENT SPECIFICATION

(11) **EP 1 463 504 B1**
(45) Date of publication and mention of the grant of the patent: **21.06.2006**
(21) Application number: 02790487.9
(22) Date of filing: 12.12.2002
(51) Int. Cl.: A61K 31/427, A61P 35/00, A61K 38/16, A61K 38/21, A61K 31/513, A61K 31/704, A61K 31/675, A61K 31/7008

(54) **COMPOSITIONS COMPRISING EPOTHILONES AND THEIR USE FOR THE TREATMENT OF THE CARCINOID SYNDROME**
PHARMAZEUTISCHE ZUBEREITUNGEN ENTHALTEND EPOTHILONE UND DEREN VERWENDUNG BEI DER BEHANDLUNG VON KARZINOID
COMPOSITIONS COMPRENANT DES EPOTHILONES ET UTILISATION DE CES DERNIERES DANS LE TRAITEMENT DU SYNDROME CARCINOIDE

(30) Priority: 13.12.2001 US 342167 P; 04.10.2002 US 415990 P
(43) Date of publication of application: 06.10.2004
(73) Proprietor: Novartis AG, 4056 Basel (CH); Novartis Pharma GmbH, 1230 Wien (AT)
(72) Inventor: ROTHERMEL, John, David, Randolph, NJ 07869 (US); RUBIN, Eric, Howard, Belle Mead, NJ 08502 (US)
(74) Representative: de Weerd, Petrus G.W.
(86) International application number: PCT/EP2002/014162
(87) International publication number: WO 2003/049734

(56) References cited:
- WO-A-01/92255
- WO-A-97/45105
- DE-A- 19 908 760

## Description

The present invention relates to the use of an epothilone of formula I as defined below, alone or in combination with at least one compound selected from the group consisting of somatostatin or a synthetic derivative thereof, interferon, 5-fluorouracil, doxorubicin, cyclophosphamide, streptozotocin and a standard anti-diarrheal, for the preparation of a medicament for the treatment of carcinoid syndrome or at least one neuroendocrine tumor; and to a combination comprising a compound of formula I as defined below and at least one compound selected from the group consisting of somatostatin or a synthetic derivative thereof, interferon, 5-fluorouracil, doxorubicin, cyclophosphamide, streptozotocin and a standard anti-diarrheal.

The epothilones, especially epothilones A, B and D, represent a new class of microtubule stabilizing cytotoxic agents (see Gerth, K. et al., J. Antibiot. 49, 560-3 (1996); or Hoefle et al., DE 41 38 042).
WO 01/92255 discloses 16-membered macrocyclic compounds of the formula (I),

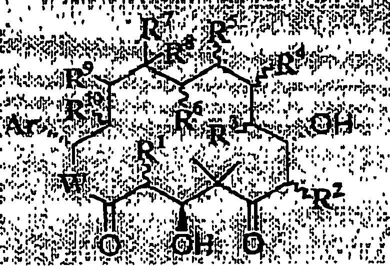

These compounds are cytotoxic agents and can be used to treat cancer and non-cancer disorders characterized by cellular hyperproliferation.

Surprisingly, it was found that epothilone derivatives of formula I 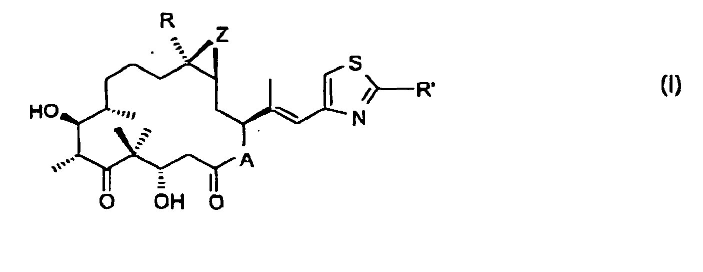 wherein A represents O or NR_{N}, wherein R_{N} is hydrogen or lower alkyl, R is hydrogen or lower alkyl, R' is methyl, methoxy, ethoxy, amino, methylamino, dimethylamino, aminomethyl or methylthio, and Z is O or a bond, produce a beneficial effect in the treatment of carcinoid syndrome and/or at least one neuroendocrine tumor.

Hence, the present invention relates to the use of a compound of formula I wherein A represents O or NR_{N}, wherein R_{N} is hydrogen or lower alkyl, R is hydrogen or lower alkyl, R' is methyl, methoxy, ethoxy, amino, methylamino, dimethylamino, aminomethyl or methylthio, and Z is O or a bond, or a pharmaceutically acceptable salt thereof, for the preparation of a medicament for the treatment of carcinoid syndrome or at least one neuroendocrine tumor.

The carcinoid syndrome is a non-metastatic disease of unknown mechanism. The term "carcinoid syndrome" as used herein relates to a disease, in particluar the manifestation of an advanced disease, the symptoms of which include cutaneous flushing, diarrhea and palpable abdominal mass or hepatomegaly. In said disease the urinary concentration of 5-hydroxyindolacetic acid (5-HIAA) typically relates directly to the tumor volume and correlates with the chance of survival. A level of > 8 mg/24 hours of 5-HIAA is a sensitive measurement in 75 % of all cases of carcinoid syndrome. Another indicator for the syndrome is an increased plasma serotonin level, in particluar a plamsa serotonin level higher than about 250, especially 350 ng/ml.

The term "neuroendocrine tumor" as used herein includes, but is not restricted to, an Islet cell tumor, gastrinoma, ViPoma and a carcinoid tumor. In one preferred embodiment of the present invention a neuroendocrine tumor is a carcinoid tumor. In another preferred embodiment of the present invention a neuroendocrine tumor is an islet cell tumor, gastrinoma or VIPoma.

The term "carcinoid tumor" as used herein relates to a neuroendocrine tumor arising from the enterochromaffin cells which cells are scattered mainly throughout the intestine and main bronchi. Peptides synthesized by carcinoid tumors include 5-hydroxytryptamine and 5-hydroxytrypthophan. Carcinoid tumor can also form metastases. The present method is especially suitable for treating metastases formed by a carcinoid tumor.

Unless stated otherwise, in the present disclosure organic radicals and compounds designated "lower" contain not more than 7, preferably not more than 4, carbon atoms.

A compound of formula I wherein A represents O, R is hydrogen, R' is methyl and Z is O is known as epothilone A; a compound of formula I wherein A represents O, R is methyl, R' is methyl and Z is O is known as epothilone B; a compound of formula I wherein A represents O, R is hydrogen, R' is methyl and Z is a bond is known as epothilone C; a compound of formula I wherein A represents O, R is methyl, R' is methyl and Z is a bond is known as epothilone D.

Epothilone derivatives of formula I wherein A represents O or NR_{N}, wherein R_{N} is hydrogen or lower alkyl, R is hydrogen or lower alkyl, R' is methyl and Z is O or a bond, and methods for the preparation of such epothilone derivatives are in particular generically and specifically disclosed in the patents and patent applications WO 93/10121, US 6,194,181, WO 98/25929, WO 98/08849, WO 99/43653, WO 98/22461 and WO 00/31247 in each case in particular in the compound claims and the final products of the working examples, the subject-matter of the final products, the pharmaceutical preparations and the claims is hereby incorporated into the present application by reference to this publications. Comprised are likewise the corresponding stereoisomers as well as the corresponding crystal modifications, e.g. solvates and polymorphs, which are disclosed therein. Epothilone derivatives of formula I, especially epothilone B, can be administered as part of pharmaceutical compositions which are disclosed in WO 99/39694, in particular epothilone B can be used formulated in polyethylene glycol 300 (PEG 300) which composition has to be pre-diluted in 0.9% sodium chloride solution to obtain a concentration of 1 mg/mL.

Epothilone derivatives of formula I wherein A represents O or NR_{N}, wherein R_{N} is hydrogen or lower alkyl, R is hydrogen or lower alkyl, R' is methoxy, ethoxy, amino, methylamino, dimethylamino, aminomethyl or methylthio, and Z is O or a bond, and methods for the preparation and administration of such epothilone derivatives are in particular generically and specifically disclosed in the patent application WO99/67252, which is hereby incorporated by reference into the present application. Comprised are likewise the corresponding stereoisomers as well as the corresponding crystal modifications, e.g. solvates and polymorphs, which are disclosed therein.

The transformation of epothilone B to the corresponding lactam is disclosed in Scheme 21 (page 31, 32) and Example 3 of WO 99/02514 (pages 48 - 50). The transformation of a compound of formula I which is different from epothilone B into the corresponding lactam can be accomplished analogously. Corresponding epothilone derivatives of formula I wherein R_{N} is lower alkyl can be prepared by methods known in the art such as a reductive alkylation reaction starting from the epothilone derivative wherein R_{N} is hydrogen.

The terms "treatment or "treating" as used herein comprises the treatment of patients having carcinoid syndrome and/or at least one neuroendocrine tumor or being in a pre-stage of said disease which treatment effects a complete response, partial response or stable disease in said patients.

The term "complete response" as used herein means in particular to the resolution of all measurable or evaluable disease.

The term "partial response" as used herein means in particular a greater than or equal to 50 % reduction in measurable or evaluable disease in the absence of progression in any particular disease site.

The term "stable disease" as used herein means in particular a less than 50 % decrease or less than 25 % increase in measurable or evaluable disease.

It will be understood that in the discussion of methods, references to the active ingredients are meant to also include the pharmaceutically acceptable salts. If these active ingredients have, for example, at least one basic center, they can form acid addition salts. Corresponding acid addition salts can also be formed having, if desired, an additionally present basic center. The active ingredients having an acid group (for example COOH) can also form salts with bases. The active ingredient or a pharmaceutically acceptable salt thereof may also be used in form of a hydrate or include other solvents used for crystallization.

In one preferred embodiment of the invention, an epothilone derivative of formula I is employed wherein A represents O, R is lower alkyl, especially methyl, ethyl or n-propyl, or hydrogen, R' is methyl, and Z is O or a bond. More preferably, an epothilone derivative of formula I is employed wherein A represents 0, R is methyl, R' is methyl and Z is O, which compound is also known as epothilone B.

In particular, the present invention relates to the use of a therapeutically effective amount of an epothilone derivative of formula I or a pharmaceutically acceptable salt thereof for treating a warm-blooded animal having carcinoid syndrome and/or at least one neuroendocrine tumor wherein said carcinoid syndrome and/or at least one neuroendocrine tumor is resistant to standard systemic chemotherapy or has demonstrated progression after radiation therapy.

The term "standard systemic chemotherapy" comprises, but is not restricted to, a method of treatment comprising administration of an agent selected from the group consisting of interferon, 5-fluorouracil, doxorubicin, cyclophosphamide and streptozotocin.

The present invention relates in particular to treating a warm-blooded animal having carcinoid syndrome and/or at least one neuroendocrine tumor wherein the warm-blooded animal has an urinary concentration of 5-hydroxyindolacetic acid of at least 8 mg per 24 hours when starting the treatment.

Treating a warm-blooded animal having carcinoid syndrome and/or at least one neuroendocrine tumor as disclosed herein can be employed as a monotherapy or in addition to an established therapy comprising, e.g., the administration of a compound selected form the group consisting of somatostatin or a synthetic derivative thereof, interferon, 5-fluorouracil, doxorubicin, cyclophosphamide, streptozotocin and/or a standard anti-diarrheal.

Hence, the present invention pertains also to a combination comprising a compound formula I wherein A represents NR_{N}, wherein R_{N} is hydrogen or lower alkyl, or, preferably, O, R is hydrogen or lower alkyl, preferably methyl, R' is methyl, methoxy, ethoxy, amino, methylamino, dimethylamino, aminomethyl or methylthio, preferably methyl, and Z is a bond or, preferably, O, and at least one compound selected from the group consisting of somatostatin or a synthetic derivative thereof, interferon, 5-fluorouracil (5-FU), doxorubicin, cyclophosphamide (also known as N,N-bis(2-chloroethyl)tetrahydro-2H-1,3,2-oxazaphosphorin-2-amine 2-oxide), streptozotocin (also known as streptozocin) and a standard anti-diarrheal, in which the active ingredients are present in each case in free form or in the form of a pharmaceutically acceptable salt and optionally at least one pharmaceutically acceptable carrier, for simultaneous, separate or sequential use, especially for such use in a method of treating a carcinoid syndrome and/or at least one neuroendocrine tumor.

A benefit of such combinations is that lower doses of the single active ingredients can be used, for example, that the dosages need not only often be smaller but are also applied less frequently, which approach can diminish the incidence of side-effects. This is in accordance with the desires and requirements of the patients to be treated.

The term "interferon" as used herein relates to a family of species-specific vertebrate proteins that confer non-specific resistance to a broad range of viral infections, affect cell proliferation and modulate immune response, e.g. interferon-α, interferon-β and interferon-γ.

The term "somatostatin" as used herein relates to a growth hormone-release inhibiting factor as described, e.g., in US 4,356,270. Synthetic derivatives of somatostatin are, e.g., those described by Brazeau et al in Endocrinology 94, 184 (1974).

The term "standard anti-diarrheal" as used herein include, but is not limited to, natural opiods, such as tincture of opium, paregoric, and codeine, synthetic opoids, such as diphenoxylate, difenoxin and loperamide, bismuth subsalicylate, octreotide, motilin antagonists and traditional antidiarrheal remedies, such as kaolin, pectin, berberine and muscarinic agents. The antidiarrheal agent is administered as a preventative measure throughout the treatment cycle or as needed when diarrhea occurs. The antidiarrheal agent is administered to prevent, control or eliminate diarrhea that is sometimes associated with the administration of epothilones, especially epothilone B, but which is also one of the symptoms of carcinoid syndrome.

Surprisingly, it was found that the diarrhea associated with the carcinoid syndrome is not enhanced strongly or not enhanced at all by a compound of formula I, despite of the fact that compounds of formula I sometimes themselves effect diarrhea.

The structure of the active ingredients identified by code nos., generic or trade names may be taken from the actual edition of the standard compendium "The Merck Index" or from databases, e.g. Patents International (e.g. IMS World Publications). The corresponding content thereof is hereby incorporated by reference. Any person skilled in the art is fully enabled to identify the active ingredients and, based on these references, likewise enabled to manufacture and test the pharmaceutical indications and properties in standard test models, both *in vitro* and *in vivo.*

A combination comprising a compound formula I wherein A represents O or NR_{N}, wherein R_{N} is hydrogen or lower alkyl, R is hydrogen or lower alkyl, R' is methyl, methoxy, ethoxy, amino, methylamino, dimethylamino, aminomethyl or methylthio, and Z is O or a bond, and at least one compound selected from the group consisting of somatostatin or a synthetic derivative thereof, interferon, 5-fluorouracil, doxorubicin, cyclophosphamide, streptozotocin and a standard anti-diarrheal, in which the active ingredients are present in each case in free form or in the form of a pharmaceutically acceptable salt and optionally at least one pharmaceutically acceptable carrier, will be referred to hereinafter as a COMBINATION OF THE INVENTION.

The COMBINATION OF THE INVENTION can be a combined preparation or a pharmaceutical composition.

The term "a combined preparation", as used herein defines especially a "kit of parts" in the sense that at least two active ingredients as defined above can be dosed independently or by use of different fixed combinations with distinguished amounts of the ingredients, i.e., simultaneously or at different time points. The parts of the kit can then, e.g., be administered simultaneously or chronologically staggered, that is at different time points and with equal or different time intervals for any part of the kit. Preferably, the time intervals are chosen such that the effect on the treated disease in the combined use of the parts is larger than the effect which would be obtained by use of only any one of the active ingredients. The ratio of the total amounts of the active ingredient 1 to the active ingredient 2 to be administered in the combined preparation can be varied, e.g., in order to cope with the needs of a patient sub-population to be treated or the needs of the single patient which different needs can be due to age, sex, body weight, etc. of the patients. Preferably, there is at least one beneficial effect, e.g., a mutual enhancing of the effect of the first and second active ingredient, in particular a synergism, e.g. a more than additive effect, additional advantageous effects, less side effects, a combined therapeutical effect in a non-effective dosage of one or both of the first and second active ingredient, and especially a synergism the first and second active ingredient.

The COMBINATION OF THE INVENTION in an amount which is jointly therapeutically can be used for treating caroinoid syndrome and/or at least one neuroendocrine tumour in a warm-blooded animal in need tereof.

The person skilled in the pertinent art is fully enabled to select relevant test models to prove the hereinbefore and hereinafter mentioned beneficial effects on carcinoid syndrome and/or at least one neuroendocrine tumor of a compound of formula I or of a COMBINATION OF THE INVENTION.

The pharmacological activity of a compound of formula I, in particular epothilone B, can be demonstrated, e.g., in a study wherein patients suffering from malignant carcinoid tumors are treated with continuous 4-week cycles (three weeks on/one week off) of epothilone B until either disease progression or unacceptable side effects occur. Response initially can be evaluated after the first two cycles, and can be based on unchanged or improved octreoscan scores, stabilization or reduction of serotonin levels, and/or stabilization or improvement in clinical symptoms. Evaluations for response can be performed, e.g., every two cycles thereafter. A complete response would be met in such a study, e.g., by a negative octreoscan, serotonin levels within normal limits or reduction of any clinical symptoms to grade 1 or less.

The pharmacological activity of a COMBINATION OF THE INVENTION may, for example, be demonstrated in an animal study or a suitable clinical study. Suitable clinical studies are, for example, open label non-randomized, dose escalation studies or placebo-controlled studies in patients with carcinoid syndrome. Such studies can in particular demonstrate a synergism produced by a COMBINATION OF THE INVENTION. The beneficial effects on carcinoid syndrome can be determined directly through the results of such studies or by changes in the study design which are known as such to a person skilled in the art. For example, one combination partner can be administered with a fixed dose and the dose of a second combination partner is escalated until the Maximum Tolerated Dosage (MTD) is reached. Alternatively, a placebo-controlled, double blind study can be conducted in order to prove the benefits of the COMBINATION OF THE INVENTION mentioned herein.

In studies using only an epothilone of formula I as well as in studies using a COMBINATION OF THE INVENTION, anti-diarrhea medication appropriate for the patient's overall condition should be administered as concomitant therapy at the first sign of abdominal cramping, loose stools or overt diarrhea. Tumor assessments can be performed by radiological techniques or if appropriate, by physical examination, e.g. subcutaneous nodules. Radiological studies must account for all lesions that were present at screening. Screening evaluations include CT scan (or MRI) of the chest, abdomen and pelvis (a bone scan, if appropriate), and any other evaluation required to establish presence of underlying disease. Further evaluations of the response to the treatment include
- an Octreoscan® ,
- the study of tumor-specific mutations and gene expression changes in tumor cells,
- the determination of the time to progression from the start of the treatment to documented disease progression, death from study indication or the date of follow-up if otherwise,
- the duration of overall response from the time that measurement criteria are met for complete response or partial response (which ever status is recorded first) until the first date that recurrent or progressive disease is objectively documented, and
- the overall survival from the date of the first administration of the epothilone to (1) the date of death or (2) the last date the patient was known to be alive.

It is one objective of this invention to provide a pharmaceutical composition comprising a quantity, which is jointly therapeutically effective against carcinoid syndrome and/or at least one neuroendocrine tumor comprising the COMBINATION OF THE INVENTION. In this pharmaceutical composition, the combination partners can be administered together, one after the other or separately in one combined unit dosage form or in two separate unit dosage forms. The unit dosage form may also be a fixed combination.

The pharmaceutical compositions for separate administration of the combination partners and for the administration in a fixed combination, i.e. a single galenical composition comprising at least two combination partners, according to the invention can be prepared in a manner known per se and are those suitable for enteral, such as oral or rectal, and parenteral administration to mammals (warm-blooded animals), including man, comprising a therapeutically effective amount of at least one pharmacologically active combination partner alone or in combination with one or more pharmaceutically acceptable carries, especially suitable for enteral or parenteral application.

Novel pharmaceutical composition contain, for example, from about 10 % to about 100 %, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se. It will be appreciated that the unit content of a combination partner contained in an individual dose of each dosage form need not in itself constitute an effective amount since the necessary effective amount can be reached by administration of a plurality of dosage units.

For example, the treatment of carcinoid syndrome and/or at least one neuroendocrine tumor according to the present invention may comprise (i) administration of a combination partner (a) in free or pharmaceutically acceptable salt form and (ii) adminstration of a combination partner (b) in free or pharmaceutically acceptable salt form, simultaneously or sequentially in any order, in jointly therapeutically effective amounts, preferably in synergistically effective amounts, e.g. in daily dosages corresponding to the amounts described herein. Furthermore, the term administering also encompasses the use of a pro-drug of a combination partner that convert *in vivo* to the combination partner as such. The instant invention is therefore to be understood as embracing all such regimes of simultaneous or alternating treatment and the term "administering" is to be interpreted accordingly.

The effective dosage of a compound of formula I or of the compounds employed in the COMBINATION OF THE INVENTION may vary depending on the particular compound or pharmaceutical composition employed, the mode of administration, the severity of the carcinoid syndrome and/or at least one neuroendocrine tumor being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of a compound of formula I or of the single active ingredients of the COMBINATION OF THE INVENTION required to prevent, counter or arrest the progress of the condition. Optimal precision in achieving concentration of the active ingredients within the range that yields efficacy without toxicity requires a regimen based on the kinetics of the active ingredients' availability to target sites.

If the the warm-blooded animal is a human, the dosage of a compound of formula I is preferably in the range of about 0.1 to 75, preferably 0.25 to 50, e.g. 2.5 or 6, mg/m² once weekly for two to four, e.g. three, weeks, followed by 6 to 8 days off in the case of an adult patient.

In one embodiment of the invention epothilone B is administered weekly in a dose that is between about 0.1 to 6 mg/m², preferably between 0.1 and 3 mg/m², e.g. 2.5 mg/m², for three weeks after an interval of one to six weeks, especially an interval of one week, after the preceding treatment. In another embodiment of the invention said epothilone B is preferably administered to a human every 18 to 24 days in a dose that is between about 0.5 and 7.5 mg/m², e.g., 5.4, 6.0 or 7.0 mg/m² epothilone B about every three weeks as a five minutes bolus injection. Alternatively, even higher doses of epothilone B, e.g. between about 8.0 mg/m² and about 18.0 mg/m², e.g. about 12 mg/m², can be administered to the patient every five or six weeks.

When the combination partners employed in the COMBINATION OF THE INVENTION are applied in the form as marketed as single drugs, their dosage and mode of administration can take place in accordance with the information provided on the package insert of the respective marketed drug in order to result in the beneficial effect described herein, if not mentioned herein otherwise.

Moreover, the present invention provides a commercial package comprising as active ingredients the COMBINATION OF THE INVENTION, together with instructions for simultaneous, separate or sequential use thereof in the treatment of carcinoid syndrome and/or at least one neuroendocrine tumor.

The present invention also provides the use of a compound of formula I as defined herein and the use of a COMBINATION OF THE INVENTION for the treatment of carcinoid syndrome and/or at least one neuroendocrine tumor and for the preparation of a medicament for the treatment of said disease.

### EXAMPLE 1

A female human patient (31 years old) experiencing 3 loose bowel movements each day, frequent nausea and anorexia presented a plasma serotonin level of 361 ng/ml and an octreotide scan showing foci of abnormal activity in the pelvis, liver, upper chest and neck. She received 2.5 mg/m² of epothilone B weekly every 3 of 4 weeks. After 6 doses of epothilone B, an octreotide scan indicated a minimal focus of activity in the pelvis alone. After further about two months under such treatment schedule, a plasma serotonin level of 62 ng/ml was determined (which is within normal limits) and an octreotide scan indicated no evidence of disease.

The observations in the described Example make it likely that the tumor burden of the patient significantly decreased as a result of the administration of epothilone B.

## Claims

1. Use of a compound of formula I 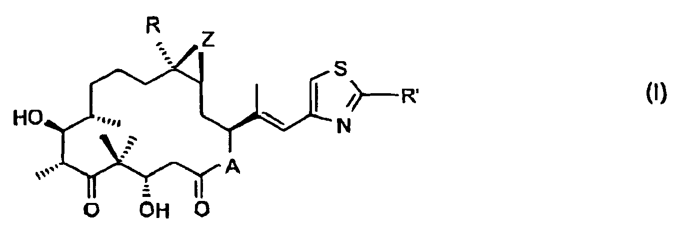 wherein A represents O or NR_{N}, wherein R_{N} is hydrogen or lower alkyl, R is hydrogen or lower alkyl, R' is methyl, methoxy, ethoxy, amino, methylamino, dimethylamino, aminomethyl or methylthio, and Z is O or a bond, wherein "lower" means not more thant 7 carbon atoms;
or a pharmaceutically acceptable salt thereof,
for the preparation of a medicament for the treatment of carcinoid syndrome or at least one neuroendocrine tumor.

2. The use according to claim 1 wherein in the compound of formula IA represents O, R is methyl, R' is methyl and Z is O

3. The use according to claim 1 or 2 wherein the carcinoid syndrome or at least one neuroendocrine tumor is resistant to standard systemic chemotherapy or has demonstrated progression after radiation therapy.

4. The use according to any one of claims 1 to 3 wherein the neuroendocrine tumor is a carcinoid tumor.

5. The use according to any one of claims 1 to 3 wherein the neuroendocrine tumor is an Islet cell tumor, gastrinoma or VIPoma.

6. Use of an epothilone derivative of formula I 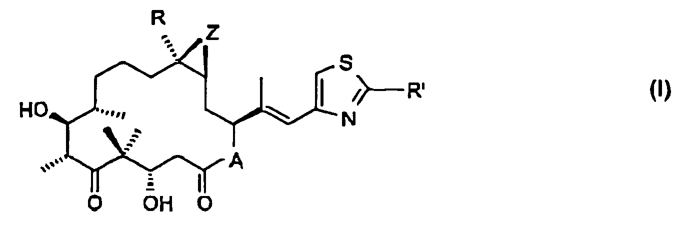 wherein A represents O or NR_{N}, wherein R_{N} is hydrogen or lower alkyl, R is hydrogen or lower alkyl, R' is methyl, methoxy, ethoxy, amino, methylamino, dimethylamino, aminomethyl or methylthio, and Z is O or a bond, wherein "lower" means not more than 7 carbon atoms,
or a pharmaceutically acceptable salt thereof for the preparation of a medicament for the treatment of a warm-blooded animal with carcinoid syndrome and/or at least one neuroendocrine tumor.

7. The use according to claim 6 wherein the warm-blooded animal is a human.

8. The use according to claim 6 or 7 in which an epothilone derivative of formula I wherein A represents O, R is methyl, R' is methyl and Z is O or a pharmaceutically acceptable salt is used.

9. The use according to any one of claims 6 to 8 wherein the carcinoid syndrome and/or at least one neuroendocrine tumor is resistant to standard systemic chemotherapy or has demonstrated progression after radiation therapy.

10. The use according to any one of claims 6 to 8 wherein the warm-blooded animal has an urinary concentration of 5-hydroxyindolacetic acid of at least 8 mg per 24 hours when starting the treatment.

11. A combination comprising a compound formula I 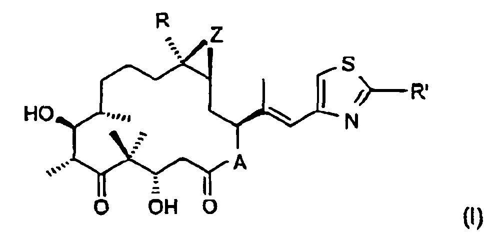 wherein A represents 0 or N%, wherein R_{N} is hydrogen or lower alkyl, R is hydrogen or lower alkyl, R' is methyl, methoxy, ethoxy, amino, methylamino, dimethylamino, aminomethyl or methylthio, and Z is O or a bond, wherein "lower" means not more than 7 carbon atoms and at least one compound selected from the group consisting of somatostatin or a synthetic derivative thereof, interferon, 5-fluorouracil, doxorubicin, cydophosphamide, streptozotocin and a standard anti-diarrheal. in which the active ingredients are present in each case in free form or in the form of a pharmaceutically acceptable salt and optionally at least one pharmaceutically acceptable carrier, for simultaneous, separate or sequential use for the treatment of carcinoid syndrome or at least one neuroendocrine tumor.

12. Combination according to claim 11 wherein in the epothilone derivative of formula I A represents O, R is methyl, R' is methyl and Z is O.

13. Combination according to any one of claims 11 or 12 which is a combined preparation or a pharmaceutical composition.

14. Use of a combination according to any one of claims 11 to 13 for the preparation of a medicament for the treatment of the carcinoid syndrome or at least one neuroendocrine tumor.

15. A pharmaceutical composition comprising a quantity, which is jointly therapeutically effective against carcinoid syndrome or at least one neuroendocrine tumor, of a combination according to claim 11 or 12 and at least one pharmaceutically acceptable carrier.

16. A commercial package comprising a combination of any one of claims 11 to 13 together with instructions for simultaneous, separate or sequential use thereof in the treatment of carcinoid syndrome or at least one neuroendocrine tumor.

## Patentansprüche

1. Verwendung einer Verbindung der Formel 1 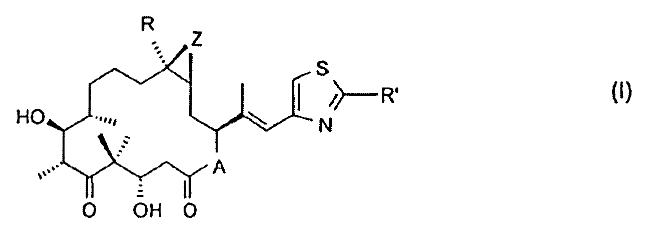 worin A für O oder NR_{N} steht, worin R_{N} für Wasserstoff oder Niederalkyl steht, R für Wasserstoff oder Niederalkyl steht, R' für Methyl, Methoxy, Ethoxy, Amino, Methylamino, Dimethylamino, Aminomethyl oder Methylthio steht und Z für O oder eine Bindung steht, worin "Nieder" nicht mehr als 7 Kohlenstoffatome meint,
oder eines pharmazeutisch annehmbaren Salzes hiervon,
zur Herstellung eines Arzneimittels zur Behandlung des carcinoiden Syndroms oder zumindest eines neuroendokrinen Tumors.

2. Verwendung nach Anspruch 1, worin in der Verbindung der Formel 1 die Brücke A für O steht, R für Methyl steht, R' für Methyl steht und Z für O steht.

3. Verwendung nach Anspruch 1 oder 2, worin das carcinoide Syndrom oder zumindest ein neuroendokriner Tumor gegen eine systemische Standardchemotherapie resistent ist oder nach einer Bestrahlungstherapie eine Progression zeigt.

4. Verwendung nach einem der Ansprüche 1 bis 3, worin der neuroendokrine Tumor ein carcinoider Tumor ist.

5. Verwendung nach einem der Ansprüche 1 bis 3, worin der neuroendokrine Tumor ein Inselzelltumor, ein Gastrinom oder ein VIPom ist.

6. Verwendung eines Epothilonderivats der Formel I 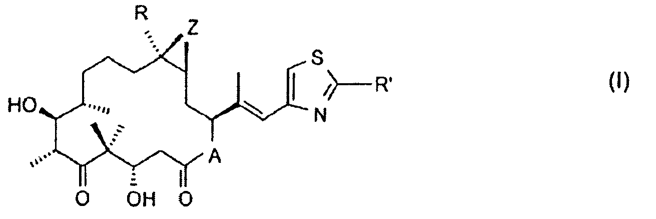 worin A für O oder NR_{N} steht, worin R_{N} für Wasserstoff oder Niederalkyl steht, R für Wasserstoff oder Niederalkyl steht, R' für Methyl, Methoxy, Ethoxy, Amino, Methylamino, Dimethylamino, Aminomethyl oder Methylthio steht und Z für O oder eine Bindung steht, worin "Nieder" nicht mehr als 7 Kohlenstoffatome meint,
oder eines pharmazeutisch annehmbaren Salzes hiervon,
zur Herstellung eines Arzneimittels zur Behandlung des carcinoiden Syndroms oder zumindest eines neuroendokrinen Tumors.

7. Verwendung nach Anspruch 6, worin der Warmblüter ein Mensch ist.

8. Verwendung nach Anspruch 6 oder 7, worin ein Epothilonderivat der Formel I, worin A für O steht, R für Methyl steht, R' für Methyl steht und Z für O steht oder ein pharmazeutisch annehmbares Salz verwendet wird.

9. Verwendung nach einem der Ansprüche 6 bis 8, worin das carcinoide Syndrom und/oder zumindest ein neuroendokriner Tumor gegenüber einer systemischen Standardchemotherapie resistent ist oder nach einer Bestrahlungstherapie eine Progression zeigt.

10. Verwendung nach einem der Ansprüche 6 bis 8, worin der Warmblüter eine Konzentration an 5-Hydroxyindolessigsäure im Harn von mindestens 8 mg pro 24 Stunden aufweist, wenn die Behandlung begonnen wird.

11. Kombination, die umfasst eine Verbindung der Formel 1 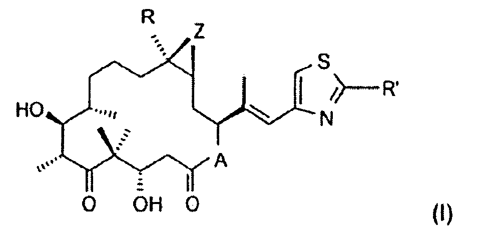 worin A für O oder NR_{N} steht, worin R_{N} für Wasserstoff oder Niederalkyl steht, R für Wasserstoff oder Niederalkyl steht, R' für Methyl, Methoxy, Ethoxy, Amino, Methylamino, Dimethylamino, Aminomethyl oder Methylthio steht und Z für O oder eine Bindung steht, worin "Nieder" nicht mehr als 7 Kohlenstoffatome meint, und mindestens eine Verbindung, die aus der Gruppe ausgewählt ist, welche besteht aus Somatostatin oder einem synthetischen Derivat hiervon, Interferon, 5-Fluoruracil, Doxorubicin, Cyclophosphamid, Streptozotocin und einem Standardantidiarrhoemittel, worin die Wirkstoffe jeweils in freier Form oder in Form eines pharmazeutisch annehmbaren Salzes vorliegen und optional zumindest einen pharmazeutisch annehmbaren Träger zur simultanen, getrennten oder sequentiellen Verwendung zur Behandlung des carcinoiden Syndroms oder zumindest eines neuroendokrinen Tumors.

12. Kombination nach Anspruch 11, worin im Epothilonderivat der Formel I die Brücke A für O steht, R für Methyl steht, R' für Methyl steht und Z für O steht.

13. Kombination nach einem der Ansprüche 11 oder 12, die ein Kombinationspräparat oder eine pharmazeutische Zusammensetzung ist.

14. Verwendung einer Kombination nach einem der Ansprüche 11 bis 13 zur Herstellung eines Arzneimittels zur Behandlung des carcinoiden Syndroms oder zumindest eines neuroendokrinen Tumors.

15. Pharmazeutische Zusammensetzung, die eine Menge einer Kombination nach Anspruch 11 oder 12, die gemeinsam therapeutisch gegen das carcinoide Syndrom oder zumindest einen neuroendokrinen Tumor wirksam ist, und zumindest einen pharmazeutischen Träger umfasst.

16. Im Handel erhältliche Packung, die eine Kombination nach einem der Ansprüche 11 bis 13 zusammen mit einem Beipackzettel zur simultanen, getrennten oder sequentiellen Verwendung hiervon zur Behandlung des carcinoiden Syndroms oder zumindest eines neuroendokrinen Tumors umfasst.

## Revendications

1. Utilisation d'un composé de formule 1 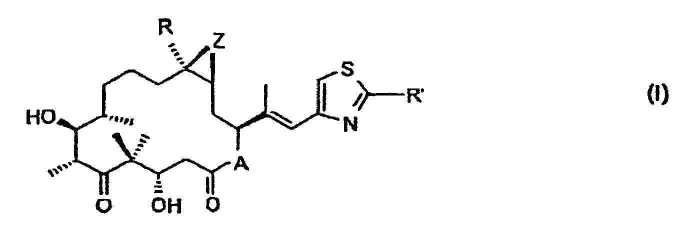 dans laquelle, A représente O ou NR_{N}, dans laquelle R_{N} est hydrogène ou alkyle inférieur, R est hydrogène ou alkyle inférieur, R' est méthyle, méthoxy, éthoxy, amino, méthylamino, diméthylamino, aminométhyle ou méthylthio, et Z est O ou une liaison, dans laquelle « inférieur » signifie pas plus de 7 atomes de carbone.
ou un de ses sels pharmaceutiquement acceptables,
pour la préparation d'un médicament destiné au traitement du syndrome carcinoïde ou d'au moins une tumeur neuroendocrine.

2. Utilisation selon la revendication 1 **caractérisée en ce que** dans le composé de formule I, A représente O, R est méthyle, R' est méthyle et Z est O.

3. Utilisation selon la revendication 1 ou 2, **caractérisée en ce que** le syndrome carcinoïde, ou au moins une tumeur neuroendocrine, résiste à une chimiothérapie systémique standard ou a fait preuve d'une évolution suite à une radiothérapie.

4. Utilisation selon l'une quelconque des revendications 1 à 3, **caractérisée en ce que** la tumeur neuroendocrine est une tumeur carcinoïde.

5. Utilisation selon l'une quelconque des revendications 1 à 3, **caractérisée en ce que** la tumeur neuroendocrine est une tumeur des cellules insulaires, un gastrinome ou un VIPome.

6. Utilisation d'un dérivé d'épothilone de formule 1 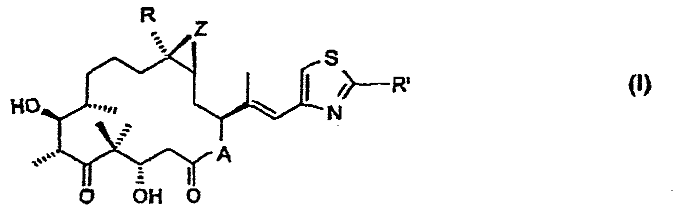 dans laquelle, A représente O ou NR_{N}, dans laquelle R_{N} est hydrogène ou alkyle inférieur, R est hydrogène ou alkyle inférieur, R' est méthyle, méthoxy, éthoxy, amino, méthylamino, diméthylamino, aminométhyle ou méthylthio, et Z est O ou une liaison, dans laquelle « inférieur » signifie pas plus de 7 atomes de carbone.
ou un de ses sels pharmaceutiquement acceptables,
pour la préparation d'un médicament destiné au traitement d'un animal à sang chaud souffrant d'un syndrome carcinoïde et/ou d'au moins une tumeur neuroendocrine.

7. Utilisation selon la revendication 6, **caractérisée en ce que** l'animal à sang chaud est un humain.

8. Utilisation selon la revendication 6 ou 7 **caractérisée en ce qu'**un dérivé d'épothilone de formule 1 dans laquelle A représente O, R est méthyle, R' est méthyle et Z est O ou un sel pharmaceutiquement acceptable est employé.

9. Utilisation selon la revendication 6 ou 8 **caractérisée en ce que** le syndrome carcinoïde et/ou au moins une tumeur neuroendocrine résistent à une chimiothérapie systémique standard ou a fait preuve d'une évolution suite à une radiothérapie.

10. Utilisation selon la revendication 6 ou 8 **caractérisée en ce que** l'animal à sang chaud présente une concentration urinaire en acide 5-hydroxyindolacétique d'au moins 8 mg par 24 heures au début du traitement.

11. Combinaison comprenant un composé de formule I 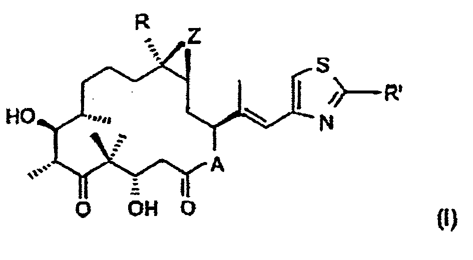 dans laquelle, A représente O ou NR_{N}, dans laquelle R_{N} est hydrogène ou alkyle inférieur, R est hydrogène ou alkyle inférieur, R' est méthyle, méthoxy, éthoxy, amino, méthylamino, diméthylamino, aminométhyle ou méthylthio, et Z est O ou une liaison, dans laquelle « inférieur » signifie pas plus de 7 atomes de carbone,
et au moins un composé sélectionné à partir du groupe composé de somatostatine ou d'un de ses dérivés synthétiques, d'interféron, de 5-fluorouracile, de doxorubicine, de cyclophosphamide, de streptozotocine et d'un anti-diarrhéique standard, dans lequel les ingrédients actifs sont présents, dans chacun des cas, sous forme libre ou sous forme d'un sel pharmaceutiquement acceptable et éventuellement au moins un véhicule pharmaceutiquement acceptable,
destinée à une utilisation simultanée, séparée ou séquentielle pour le traitement d'un syndrome carcinoïde et/ou d'au moins une tumeur neuroendocrine.

12. Combinaison selon la revendication 11, **caractérisée en ce que**, dans le dérivé d'épothilone de formule I, A représente O, R est méthyle, R' est méthyle et Z est O.

13. Combinaison selon la revendication 11 ou 12 qui est une préparation combinée ou une composition pharmaceutique.

14. Utilisation d'une combinaison selon l'une quelconque des revendications 11 à 13 dans la fabrication d'un médicament destiné au traitement du syndrome carcinoïde et/ou d'au moins une tumeur neuroendocrine.

15. Composition pharmaceutique comprenant une quantité, qui est également thérapeutiquement efficace à l'encontre du syndrome carcinoïde ou d'au moins une tumeur neuroendocrine, d'une combinaison selon la revendication 11 ou 12 et au moins un véhicule pharmaceutiquement acceptable.

16. Conditionnement commercial comprenant une combinaison selon l'une quelconque des revendications 11 à 13, de pair avec des instructions permettant une administration simultanée, séparée ou séquentielle, destiné au traitement du syndrome carcinoïde ou d'au moins une tumeur neuroendocrine.
